# EUROPEAN PATENT SPECIFICATION

(11) **EP 2 504 780 B1**
(45) Date of publication and mention of the grant of the patent: **22.05.2019**
(21) Application number: 10833672.8
(22) Date of filing: 26.11.2010
(51) Int. Cl.: G06F 17/30, G06F 21/00, G06Q 50/00, A61B 5/00, G06F 19/00

(54) **SYSTEM COMPRISING DATABASE AND SAFETY DEVICE**
SYSTEM MIT EINER DATENBANK UND EINER SICHERHEITSVORRICHTUNG
SYSTÈME COMPRENANT UNE BASE DE DONNÉES ET UN DISPOSITIF DE SÉCURITÉ

(30) Priority: 27.11.2009 SE 0950910
(43) Date of publication of application: 03.10.2012
(73) Proprietor: Brifffid AB, 181 43 LIDINGÖ (SE)
(72) Inventor: Bergstedt, Britta, 181 43 Lidingö (SE)
(74) Representative: Groth & Co. KB
(86) International application number: PCT/SE2010/051307
(87) International publication number: WO 2011/065910

(56) References cited:
- WO-A1-01/71469
- WO-A1-2008/043341
- WO-A1-2009/071194
- WO-A2-2006/019623
- WO-A2-2008/027626
- US-A1- 2008 027 752
- US-A1- 2008 065 419
- US-A1- 2008 133 273
- US-A1- 2008 172 737
- US-A1- 2008 177 569
- US-A1- 2008 262 868
- US-A1- 2009 037 224
- US-B1- 7 278 028

## Description

### TECHNICAL FIELD

The present invention relates to a system comprising a database, where in the database, there is stored personal information regarding at least one individual, which information comprises information regarding said individual's state of health. The system also comprises a safety device adapted to protect at least parts of the information against unauthorized access.

### PRIOR ART

It is previously known to store medical records in a database where the responsible care provider stores specific information about the state of health of an individual. This information is accessible to the responsible nursing staff, and the individual him-/herself may be granted access to the information about him-/herself if this is asked for.

It is also known that an individual uses a so-called patient's card, a plastic card including name, personal code number and address data used in the care sector. It simplifies the administrative work for the nursing staff and minimizes the risk of personal data becoming incorrect. The patient's card is embossed free of charge at the patient information desk of the hospitals and is an important stage not only upon the admission but all through the "care chain".

Different care providing institutions with which an individual may get into contact use different systems to identify the individual and store information about the individual. Accordingly, in certain cases, an individual is identified by a patient's card and in other cases by some other kind of identification. The respective care provider uses its own databases to store medical records including information about the individual. This entails that different hospitals among themselves have different records about one and the same individual. Care providers in public as well as private medical services and other institutions that an individual is contacting, such as hospitals, dentists, care centres, optical and audiology clinics, opticians, pharmacies and different therapists, have all the their own systems to store information about their respective patients, where one and the same individual may be present in one or more of these different systems.

Publication US 2009/0037224 A1 discloses a system where a user carries an electronic device upon which electronic medical records are aggregated from multiple electronic repositories and displayed as a single set of records. Emergency medical service providers may be able to access medical records for a patient using the electronic device after being authenticated as a valid/licensed medical services provider.

It should also be mentioned that different systems are known whereby the identity of a user or an individual can be established. These may roughly be divided into two categories, one category where the user or the individual carries something that can be used to show his/her identity, such as a magnetic card, a smart card, a bar-code, an RFID transmitter, an inductive transmitter, or a USB key, whereby it is possible to establish the identity of the carrier. Another category is that the user or the individual is identified by biometric information that is unique to the user or the individual.

### SUMMARY OF THE PRESENT INVENTION

### Problems

There is a technical problem to be able to afford integrated information about the state of health of an individual where a care provider can be given a complete picture of the actions of different care providers, which complete picture may be crucial to be able to afford adequate aid in a specific situation.

There is a technical problem to afford the individual a security that personal information is handled in a proper way, that the information only is accessible to authorized users, and that required information is accessible in case of special needs, such as upon acute illnesses or accidents when the individual maybe is not close to the care provider.

There is also a technical problem to afford access to general information about the state of health of an individual for a certain category of users, where this general information concerns information that is required to provide adequate help in public medical service or in connection with an acute situation, and to afford access to specific information for another category of users, where this specific information concerns confidential information regarding a specific health care action.

There is a problem to prevent abuse or unauthorized access to the information, as well as to prevent anyone from changing, deleting or adding information about an individual without this being made in a way so that the individual or authorized users can view what has been made, when it was made, and who made it.

There is a problem to allow the individual to get access in a simple way to the general as well as the specific information that has been stored regarding him-/herself, and simultaneously to prevent said individual from altering the information about him-/herself in an undue way.

There is also a problem to make accessible the information to a user in several different places, such as at various care institutions or even outside in the field at scenes of accidents or emergency areas.

There is a technical problem to make the information accessible to a user that does not know the individual if the individual is not contactable, such as at a scene of an accident where an individual is found and is unconscious.

### Solution

The invention provides a solution to the problems defined above by means of a system according to claim 1.

With the purpose of solving one or several of the above problems, and on the basis of prior art such as it has been shown above and the indicated technical field, the present invention teaches that a database according to the invention is divided into a first storage space and a second storage space, and that the safety device is adapted to grant an authorized user access to the first storage space according to a first safety level and to the second storage space according to a second safety level.

In the first storage space, there is stored general information about the individual's state of health, and in the second storage space, there is stored specific information about the individual's state of health. The safety device is adapted to demand a first authorization key belonging to the individual in combination with a second authorization key belonging to the authorized user in order for access to the first storage space to be granted to the authorized user.

When all information about the individual is integrated in a common database, an authorized user can accordingly be granted access to a general picture of the different care actions the individual has been subjected to, which may be crucial to how continued care should be offered in a way that is best for the individual.

The present invention teaches that the general information concerns information that is required to provide adequate help in an acute situation, such as the individual's personal data, blood group, diseases that may require fast actions, mental or physical handicap, heart disease, diabetes, allergies, epilepsy, impaired vision and/or hearing as well as vitally important medication, and that the first safety level is adapted to grant an authorized user from public or private medical service or immediate assistance corps, such as staff from the rescue services agency, ambulance services, fire fighting services, police force, pharmacies, dentists, opticians, receptions in emergency wards, optical and audiology clinics, care institutions and primary care, access to this general information.

This solves the existing problems of being able to take into consideration the general condition of an individual in connection with an acute situation or of easily being able to get a comprehensive picture of the individual's general state of health when the individual gets into contact with public medical service.

The present invention teaches further that the specific information concerns confidential information regarding a specific health care action, and that the second safety level is adapted to grant registered staff in the care sector access to specific information about the individual regarding the special action this very user works with, where registered staff, for instance, may be caring staff such as a physician, a nurse, a dentist, an optician, or a registered therapist. Registered staff may also consist of non-nursing staff that still may need access to data in the specific information, such as an officer at the regional social insurance office.

The present invention teaches that the safety device comprises means for reading and checking the first and second authorization key.

With the purpose of making accessible the information of the database in several different and geographically scattered places, the present invention teaches that the safety device comprises a plurality of different means, which means are distributed and geographically placed to be easily accessible to different authorized users.

With the purpose of securely establishing that the information in the database is not incorrectly changed or deleted, or that information is incorrectly added, the present invention teaches that the safety device is adapted to determine a first degree of authorization, which is adapted to give an authorized user an authorization to read information from the database, a second degree of authorization, which is adapted to give an authorized user authorization to write information to the database, as well as a third degree of authorization, which is adapted to give an authorized user authorization to remove information from the database, and that an authorized user can be assigned access to the information in the database according to a suitable combination of the first and second safety level and the first, second and third degree of authorization, respectively.

With the intention to afford a possibility of viewing who has made changes in the information, when changes were made and what changes were being made, the present invention teaches that the system comprises a log, and that each access of the database, and each action taken upon such an access, is recorded in the log together with the user's identity as well as the instant of time of the access.

In order to give an individual the possibility of viewing which information is stored regarding him-/herself, the present invention teaches that the safety device is adapted to grant the individual access to the first and second storage space by means of the first authorization key. Such an access for the individual is assigned at least according to the first degree of authorization, i.e., the right to read information.

An authorization key may consist of different items whereby an individual and a user can identify him-/herself. Accordingly, it comprises a means that is available to read and check the first and second authorization key, an interface for the communication with an authorization key, such as a keyset, a touch panel, a reader of magnetic cards or smart cards, a reader of bar-codes, an RFID receiver, an inductive receiver, or an interface for the communication with a USB key, and the first and/or second authorization key may accordingly consist of something an individual or a user possesses, such as a personal code, a magnetic card, a smart card, a bar-code, an RFID transmitter, an inductive transmitter, or a USB key.

According to the present invention, it is also possible that the means that is available to read and check the first and second authorization key comprises an interface for the reading of biometric information and the provision of biometric identification, such as a reader of finger prints, hand prints, eye recognition, ear recognition, face recognition, or voice recognition, and that the first and/or second authorization key are/is a biometrically unique feature belonging to the individual or the user.

Another type of biological unique information is the DNA of an individual, which in certain cases, such as in accidents or catastrophes, may be the only way of identifying an individual, so it is possible that the means that is available to read and check the first authorization key comprises analysis tools for DNA, and that the first authorization key is the individual's DNA.

It is also possible that the safety device comprises means that are mobile, and that these means are adapted to be carried in emergency vehicles or rescue vehicles to be usable at scenes of accidents or emergency areas and in such a way grant access to information about individuals that may need help directly on a scene of an accident.

With the intention to afford a possibility of giving an authorized user access to the general information in a situation where it is difficult or impossible to establish communication with the database, the present invention teaches that the means may comprise a third storage space, and that a copy of the general information stored in the first storage space and belonging to the respective individual is stored in the third storage space.

This makes it possible to grant an authorized user access to the general information via the third storage space in a situation where it is difficult or impossible to establish communication with the database.

In order to afford a possibility of keeping the third storage space updated, the present invention teaches that the third storage space is adapted to be synchronized with the first storage space upon a contact between the means and the database.

The first authorization key should preferably be carried by the individual all the time, and the present invention teaches different kinds of first authorization keys that easily are brought and carried by the individual without major effort. In the same way, it may be important that also the second authorization key easily can be carried by a user.

Accordingly, the present invention teaches that the first and/or second authorization key are/is inserted by operation in the individual's or the user's body.

It is also possible that the first and/or second authorization key are/is a piece of jewellery or, for instance, a tattoo.

### Advantages

The advantages that foremost may be associated with a system according to the present invention are that the system according to the invention affords integrated information about the state of health of an individual, which means that a care provider can be granted access to crucial information that is required to be able to provide a correct caring action.

Even if the information is integrated, the system only allows that authorized users are granted access to the information, and different users can be given different authorization so that only required information should be accessible to the respective user.

The general information is accessible everywhere where means for reading the individual's authorization key are available, which means that also in acute situations at scenes of accidents or emergency areas, ambulance service staff or rescue services staff can be granted access to essential information about the medical background and health of the victims, and this even if the individual is unconscious. Accordingly, the present invention can save lives by crucial information being easily accessible in critical situations.

A further advantage is that the present invention gives the possibility of fast and easily identifying deceased individuals by reading the individual's authorization key.

The present invention also grants the individual access to his/her own stored information, which gives a possibility of his/her own checking of the information to avoid mistake and prevent errors.

The present invention also affords the individual a freedom to make his/her own choice of care provider and still know that this care provider has access to possible information of earlier care actions as well as information about the current state of health as the basis for the care actions that should be carried out.

By the present invention, a possibility of compiling a historical account of the medical and care record of an individual is afforded. Accordingly a historical account of, for instance, the individual's vaccinations may be readily compiled. It is also possible to compile other historical accounts, such as the accumulated exposure to x-rays of an individual or an accumulated intake of a medical preparation.

### BRIEF DESCRIPTION OF THE DRAWINGS

A system having the properties associated with the present invention will now be described in more detail for the purpose of exemplifying, reference being made to the accompanying drawing, wherein:
Figure 1 shows schematically a system comprising a database and a safety device, and
Figure 2 shows schematically a system according to the invention.

### DETAILED DESCRIPTION OF PREFERRED EMBODIMENTS

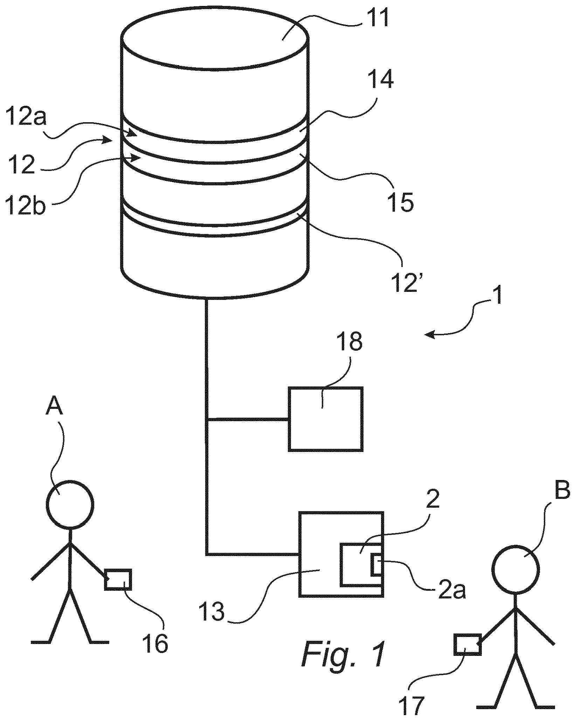

In the following, the present invention will be described with a reference to Figure 1 showing a system 1 comprising a database 11 in which personal information 12 regarding at least one individual A is stored, where the information 12 comprises information regarding the individual A's state of health.

It should be appreciated that even if the present invention in the following many times will be described with an individual A, the invention is adapted to handle information related to a large number of individuals where the advantages of the invention increase with the number of individuals and where the number of individuals only is limited by the size of accessible storage spaces in the database and the capacity of the database management used of handling great amounts of information.

A system 1 according to the present invention also comprises a safety device 13 adapted to protect at least parts of the information 12 against unauthorized access.

The present invention teaches particularly that the database 11 is divided into a first storage space 14 and a second storage space 15, and that the safety device 13 is adapted to grant an authorized user B access to the first storage space 14 according to a first safety level and to the second storage space 15 according to a second safety level.

It should be appreciated that even if the present invention in the following many times will be described with a user B, the invention is adapted to handle a large number of users and their respective authorizations where the advantages of the invention can be considered to increase with the number of users.

In the first storage space 14, there is stored general information 12a about the individual A's state of health, and in the second storage space 15, there is stored specific information 12b about the individual A's state of health. The safety device 13 is adapted to demand a first authorization key 16 belonging to the individual A in combination with a second authorization key 17 belonging to the authorized user B in order for access to the first storage space 14 to be granted to the authorized user B.

The general information 12a concerns information that is required to provide adequate help in an acute situation. Such information may be the individual A's personal data, blood group, diseases that may require fast actions, mental or physical handicap, heart disease, diabetes, allergies, epilepsy, impaired vision and/or hearing as well as vitally important medication. The first safety level is adapted to grant an authorized user B from public medical service or immediate assistance corps, such as staff from the rescue services agency, ambulance services, fire fighting services, police force, pharmacies, dentists, opticians, receptions in emergency wards, optical and audiology clinics, care institutions and primary care, access to the general information 12a.

The specific information 12b concerns confidential information regarding a specific health care action, and the second safety level is adapted to grant users B, which are registered staff in the care sector, access to specific information about the individual regarding the special action this very user works with, where registered staff, for instance, may be a physician, a nurse, a dentist, an optician, a registered therapist, a pharmacist, or an officer at the regional social insurance office.

It should be appreciated that different users may, according to his/her respective authorization, be granted access to different parts of the general information 12a and the specific information 12b, the information 12 is accordingly selectively accessible according to authorization. For instance, a pharmacist can only read prescriptions but is not granted access to the entire case sheet of an individual. Officers at, for instance, an insurance company have only access to information requisite for them. A physician may be given further authorization to be able to view information regarding other users' caring actions, thereby being able to adapt his/her own action to what has been made in other circumstances.

It should be appreciated that the present invention is not limited to how "registered staff' is defined, since the one who is to be considered to be a user that should get access to the system according to the invention may vary from country to country depending on local legislation. In some countries, complementary or alternative forms of treatment are accepted, and therapists that act within these forms of treatment may then, for instance, be registered staff. The important thing is to protect the individuals' integrity by the information only being made accessible to users that ought to have access to it.

The present invention teaches that an authorized user B also may be person or a company that has got admission or commission from the individual A, such as an insurance company or physician that works in an insurance company.

According to a proposed embodiment, it is possible to make accessible the information 12 in anonymous form 12' regarding the individual's identity, i.e., the information about the individuals is accessible but without the respective individual can be identified and associated with the information. Such anonymous information 12' may be very valuable in other contexts than in caring actions of the individual A, such as for research and development, statistical compilations or for evaluation of medicines, methods of treatment, or other caring actions, particularly since the information 12 and accordingly also the anonymous information 12' is updated continuously in connection with the individuals' contact with different medical care institutions, which makes that new and fresh information is made directly accessible in anonymous form 12' for research and development, statistical compilations and evaluations.

It is suggested that the safety device 13 is adapted to determine a first degree of authorization, which is adapted to give an authorized user B an authorization to read information from the database 11, a second degree of authorization, which is adapted to give an authorized user B authorization to write information to the database 11, as well as a third degree of authorization, which is adapted to give an authorized user B authorization to remove information from the database 11, and the safety device 13 is adapted to assign an authorized user B access to the information 12 in the database 11 according to a suitable combination of the first and second safety level and the first, second and third degree of authorization, respectively.

The present invention teaches that the system 1 comprises a log 18, in which each access of the database 11, and each action taken upon such an access, is recorded together with the user B's identity as well as the instant of time of the access.

It is possible to adapt the safety device 13 to grant the individual A access to the first and second storage space 14, 15 by means of the first authorization key 16, in which way the individual him-/herself can get access to the information available about him/her.

Such an access is assigned at least according to the first degree of authorization, i.e., the authorization to read information. Such an access can, for instance, be used to allow the individual A to get reminded about medication, vaccinations or next visit to medical care.

If the individual A also is granted access to the database 11 according to the second degree of authorization, i.e., the authorization to write information, such an access and authorization may, for instance, be used to allow the individual A to make his/her own notes about his/her state of health and accordingly create a historical account of his/her state of health, where a user B at a later stage can be granted access to such personal notes made by the individual A.

The present invention teaches that the safety device 13 comprises means 2 to read and check the first and second authorization key 16, 17.

Such means 2 comprises an interface 2a for the communication with an authorization key 16, 17.

Such an interface 2a may be a keyset, a touch panel, a reader of magnetic cards or smart cards, a reader of bar-codes, an RFID receiver, an inductive receiver, or an interface for the communication with a USB key.

Accordingly, the first and/or second authorization key 16, 17 may consist of something an individual A or user B possesses and that can be used to communicate with a means 2 according to the invention. Hence, an authorization key 16, 17 may, for instance, be a personal code, a magnetic card, a smart card, a bar-code, an RFID transmitter, an inductive transmitter, or a USB key.

It is also possible that a means 2 according to the invention comprises an interface 2a for the reading of biometric information and the provision of biometric identification, such as a reader of finger prints, hand prints, eye recognition, ear recognition, face recognition, or voice recognition, and that the first and/or second authorization key 16, 17 are/is a biometrically unique feature belonging to the individual A or the user B.

For instance, it is possible that the means 2 comprises analysis tools for DNA, and that the first authorization key is the individual's DNA.

It should be appreciated that a means 2 may comprise different interfaces whereby the means 2 can communicate with different types of authorization keys.

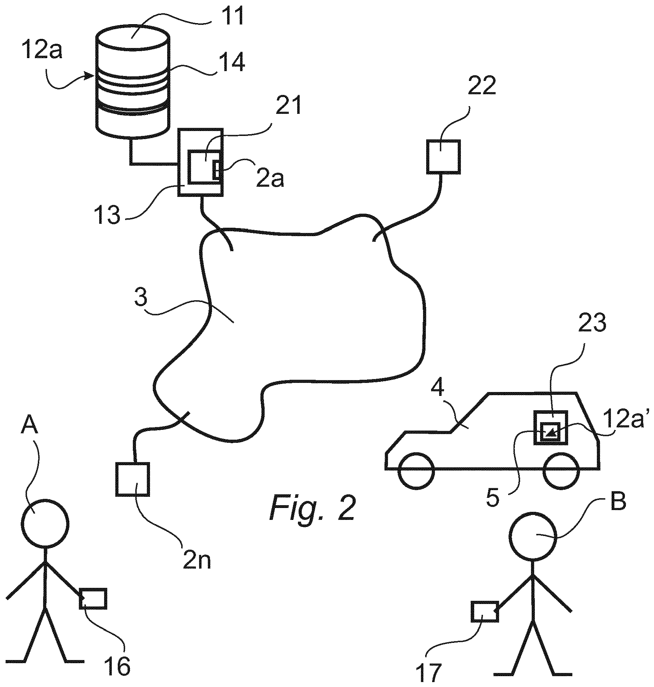

Figure 2 shows a system according to the invention, where the safety device 13 comprises a plurality of different means 21, 22, 23, ..., 2n, which may be distributed and geographically placed to be easily accessible to the authorized user B. These means may be mutually different regarding interfaces 2a for the communication with an authorization key to allow a communication with different types of authorization keys.

The distributed means 21, 22, 23, ..., 2n may be adapted to communicate with the database 11 in various ways. One part may communicate via connection to the Internet 3, others may communicate via local networks, telecommunication, radio communication or in other ways. The invention is not limited to how the different distributed means 21, 22, 23, .., 2n communicate with the database 11.

It should also be mentioned that a means 23 according to the invention may be mobile, where the means 23 is adapted to be carried in an emergency vehicle or rescue vehicle 4 in order to be accessible and useable at scenes of accidents or emergency areas.

It is suggested that the means 23 may comprise a third storage space 5, and that a copy 12a' of the general information 12a stored in the first storage space 14 and belonging to the respective individual A is stored in the third storage space 5.

This means that an authorized user B can be granted access to the copy 12a' of the general information 12a via the third storage space 5 in a situation where it is difficult or impossible to establish communication with the database 11, which may be the case at inaccessible scenes of accidents or in connection with natural disasters. When the copy 12a' of the general information 12a is accessible locally, authorized users B can still gain access to the general information 12a of an individual by the individual's first authorization key 16 and the user's second authorization key 17 in spite of the circumstances maybe making that no communication can be established with the database 11.

It is suggested that the third storage space 5 is adapted to be synchronized with the first storage space 14 upon a contact between the means 23 and the database 11.

There are different ways to carry an authorization key 16, 17, and in order for the individual A or the user B always to be able to carry his/her authorization key 16, 17, the present invention teaches that the first and/or second authorization key 16, 17 may be inserted by operation in the individual A's or the user B's body.

It is also possible that the first and/or second authorization key 16, 17 are/is a piece of jewellery or a tattoo.

The invention is of course not limited to the embodiments given above as examples but may be subjected to modifications within the scope of the subsequent claims.

## Claims

1. System (1) comprising a database (11), where in the database, there is stored personal information (12) regarding several individuals (A), where said information (12) comprises information regarding said individual's state of health, as well as a safety device (13) adapted to protect at least parts of said information (12) against unauthorized access, where said database (11) is divided into a first storage space (14) and a second storage space (15), where said safety device (13) is adapted to grant authorized users (B) access to said first storage space (14) according to a first safety level and to said second storage space (15) according to a second safety level, where in said first storage space (14), there is stored general information (12a) about each of said individual's (A) state of health, where in said second storage space (15), there is stored specific information (12b) about each of said individual's (A) state of health, where said safety device (13) is adapted to demand a first authorization key (16) belonging to said individual (A) in combination with a second authorization key (17) belonging to said authorized user (B) in order for access to said first storage space (14) to be granted to said authorized user (B), where said general information (12a) concerns information that is required to provide adequate help in an acute situation, where said first safety level is adapted to grant an authorized user from public medical service or immediate assistance corps access to said general information (12a), where said specific information (12b) concerns confidential information regarding a specific health care action, where said second safety level is adapted to grant registered staff in the care sector access to specific information about said individual regarding the special action this very user works with, where
said safety device (13) comprises means (2) for reading and checking said first and second authorization keys (16, 17), where said means (2) comprises an interface (2a) for the communication with an authorization key (16, 17), where said first and/or second authorization key (16, 17) are/is something an individual or a user possesses, **characterized in that**
said safety device (13) comprises a plurality of different reading and checking means (21, 22, 23, ..., 2n), each being configured for reading and checking said first and second authorization keys (16, 17),
and **in that** said safety device (13), in response to a first authorization key (16) belonging to any individual (A) in combination with a second authorization key (17) belonging to any authorized user (B), is configured to grant access to said general information (12a) from said first storage space (14) concerning the individual (A) to which said first authorization key belongs,
and that said means are distributed and geographically placed to be easily accessible to said authorized user (B).

2. System according to claim 1, **characterized in that** said safety device (13) is adapted to determine a first degree of authorization, which is adapted to give an authorized user (B) an authorization to read information from said database (11), a second degree of authorization, which is adapted to give an authorized user (B) authorization to write information to said database (11), as well as a third degree of authorization, which is adapted to give an authorized user (B) authorization to remove information from said database (11), that an authorized user (B) can be assigned access to the information in said database (11) according to a suitable combination of said first and second safety level and said first, second and third degree, respectively, of authorization, that said system (1) comprises a log (18), and that each access of said database (11), and each action taken upon such an access, is recorded in said log (18) together with said user's (B) identity as well as the instant of time of the access.

3. System according to any one of the preceding claims, **characterized in that** said safety device is adapted to grant said individual access to said first and second storage space (14, 15) by means of said first authorization key (16), and that said access is assigned at least according to said first degree of authorization.

4. System according to any one of the preceding claims, **characterized in that** said means (2) comprises an interface (2a) for the reading of biometric information and the provision of biometric identification, and that said first and/or second authorization key (16, 17) are/is a biometrically unique feature belonging to said individual or user.

5. System according to any one of the preceding claims, **characterized in that** said means (2) comprises analysis tools for DNA, and that said first authorization key is said individual's DNA.

6. System according to claim 1, **characterized in that** said means (23) is mobile, and that said means (23) is adapted to be carried in emergency vehicles or rescue vehicles (4).

7. System according to claim 6, **characterized in that** said means (23) comprises a third storage space (5), that a copy (12a') of the general information (12a) stored in said first storage space (14) and belonging to the respective individual (A) is stored in said third storage space (5), that an authorized user (B) can be granted access to said general information (12a) via said third storage space (5) in a situation where it is difficult or impossible to establish communication with said database (11), and that said third storage space (5) is adapted to be synchronized with said first storage space (14) upon a contact between said means (23) and said database (11).

8. System according to any one of claims 1 to 3, **characterized in that** said first and/or second authorization key (16, 17) are/is inserted by operation in said individual's (A) or user's (B) body.

9. System according to any one of claims 1 to 3, **characterized in that** said first and/or second authorization key (16, 17) are/is a piece of jewellery.

10. System according to any one of claims 1 to 3, **characterized in that** said first and/or second authorization key (16, 17) are/is a tattoo.

## Patentansprüche

1. System (1), das eine Datenbank (11), in der persönliche Daten (12) von einigen Personen (A) gespeichert sind, wobei die Daten (12) Daten bezüglich des Gesundheitszustands der Person umfassen, und eine Sicherheitseinrichtung (13), die ausgelegt ist, mindestens Teile der Daten (12) gegen unberechtigten Zugriff zu schützen, umfasst, wobei die Datenbank in einen ersten Speicherbereich (14) und einen zweiten Speicherbereich (15) unterteilt ist und die Sicherheitseinrichtung (13) ausgelegt ist, berechtigten Anwendern (B) gemäß einer ersten Sicherheitsstufe Zugriff auf den ersten Speicherbereich (14) und gemäß einer zweiten Sicherheitsstufe Zugriff auf den zweiten Speicherbereich (15) zu gewähren, wobei im ersten Speicherbereich (14) allgemeine Daten (12a) über den Gesundheitszustand jeder Person (A) gespeichert sind und im zweiten Speicherbereich (15) konkrete Daten (12b) über den Gesundheitszustand jeder Person (A) gespeichert sind, die Sicherheitseinrichtung (13) ausgelegt ist, einen ersten Berechtigungsschlüssel (16), der der Person (A) gehört, in Kombination mit einem zweiten Berechtigungsschlüssel (17), der dem berechtigen Anwender (B) gehört, zu verlangen, damit dem berechtigten Anwender (B) Zugriff zum ersten Speicherbereich (14) gewährt werden kann, die allgemeinen Daten (12a) Daten betreffen, die erforderlich sind, in einer akuten Situation angemessene Hilfe bereitzustellen, die erste Sicherheitsstufe ausgelegt ist, einem berechtigten Anwender aus dem öffentlichen Sanitätsdienst oder einem Soforthilfekorps Zugriff auf die allgemeinen Daten (12a) zu gewähren, die konkreten Daten (12b) vertrauliche Daten bezüglich einer bestimmten Maßnahme zur medizinischen Versorgung betreffen und die zweite Sicherheitsstufe ausgelegt ist, registriertem Personal im Pflegebereich Zugriff auf konkrete Daten der Person hinsichtlich der speziellen Maßnahme, an der der bestimmte Anwender arbeitet, zu gewähren, wobei
die Sicherheitseinrichtung (13) ein Mittel (2) zum Lesen und Prüfen des ersten und des zweiten Berechtigungsschlüssels (16, 17) umfasst, das Mittel (2) eine Schnittstelle (2a) für die Kommunikation mit einem Berechtigungsschlüssel (16, 17) umfasst und der erste und/oder der zweite Berechtigungsschlüssel (16, 17) etwas ist, das eine Person oder ein Anwender besitzt, **dadurch gekennzeichnet, dass**
die Sicherheitseinrichtung (13) mehrere verschiedene Lese- und Prüfmittel (21, 22, 23, ..., 2n) umfasst, die jeweils zum Lesen und Prüfen des ersten und des zweiten Berechtigungsschlüssels (16, 17) konfiguriert sind,
die Sicherheitseinrichtung (13) konfiguriert ist, als Antwort auf einen ersten Berechtigungsschlüssel (16), der einer Person (A) gehört, in Kombination mit einem zweiten Berechtigungsschlüssel (17), der einem berechtigten Anwender (B) gehört, Zugriff auf die allgemeinen Daten (12a) aus dem ersten Speicherbereich (14), die die Person (A), der der erste Berechtigungsschlüssel gehört, betreffen, zu gewähren, und
die Mittel verteilt sind und geographisch derart angeordnet sind, dass sie für den berechtigten Anwender (B) leicht zugänglich sind.

2. System nach Anspruch 1, **dadurch gekennzeichnet, dass** die Sicherheitseinrichtung (13) ausgelegt ist, einen ersten Berechtigungsgrad, der ausgelegt ist, einem berechtigten Anwender (B) eine Berechtigung zu geben, Daten aus der Datenbank (11) zu lesen, einen zweiten Berechtigungsgrad, der ausgelegt ist, einem berechtigen Anwender (B) eine Berechtigung zu geben, Daten in die Datenbank (11) zu schreiben, sowie einen dritten Berechtigungsgrad, der ausgelegt ist, einem berechtigten Anwender (B) eine Berechtigung zu geben, Daten aus der Datenbank (11) zu entfernen, zu bestimmen, dass einem berechtigten Anwender (B) Zugriff auf die Daten in der Datenbank (11) gemäß einer geeigneten Kombination der ersten und der zweiten Sicherheitsstufe und dem ersten, dem zweiten bzw. dem dritten Berechtigungsgrad zugewiesen werden kann, dass das System (1) ein Protokoll (18) umfasst und dass jeder Zugriff auf die Datenbank (11) und jede Maßnahme, die nach einem derartigen Zugriff ergriffen wird, gemeinsam mit der Identität des Anwenders (B) sowie dem Zeitpunkt des Zugriffs im Protokoll (18) aufgezeichnet wird.

3. System nach einem der vorhergehenden Ansprüche, **dadurch gekennzeichnet, dass** die Sicherheitseinrichtung ausgelegt ist, der Person mittels des ersten Berechtigungsschlüssels (16) Zugriff auf den ersten und den zweiten Speicherbereich (14, 15) zu gewähren und dass der Zugriff mindestens gemäß dem ersten Berechtigungsgrad zugewiesen wird.

4. System nach einem der vorhergehenden Ansprüche, **dadurch gekennzeichnet, dass** das Mittel (2) eine Schnittstelle (2a) zum Lesen biometrischer Daten und die Bereitstellung einer biometrischen Identifizierung umfasst und dass der erste und/oder der zweite Berechtigungsschlüssel (16, 17) biometrisch eindeutige Merkmale sind, die der Person oder dem Anwender gehören.

5. System nach einem der vorhergehenden Ansprüche, **dadurch gekennzeichnet, dass** das Mittel (2) DNA-Analysewerkzeuge umfasst und dass der erste Berechtigungsschlüssel die DNA der Person ist.

6. System nach Anspruch 1, **dadurch gekennzeichnet, dass** das Mittel (23) mobil ist und dass das Mittel (23) ausgelegt ist, in Einsatzfahrzeugen oder Rettungsfahrzeugen (4) mitgeführt zu werden.

7. System nach Anspruch 6, **dadurch gekennzeichnet, dass** das Mittel (23) einen dritten Speicherbereich (5) umfasst, dass eine Kopie (12a') der allgemeinen Daten (12a), die im ersten Speicherbereich (14) gespeichert sind und der entsprechenden Person (A) gehören, im dritten Speicherbereich (5) gespeichert ist, dass einem berechtigten Anwender (B) mittels des dritten Speicherbereiches (5) in einer Situation, in der es schwierig oder unmöglich ist, eine Kommunikation mit der Datenbank (11) aufzubauen, Zugriff auf die allgemeinen Daten (12a) gewährt werden kann und dass der dritte Speicherbereich (5) ausgelegt ist, nach einem Kontakt zwischen dem Mittel (23) und der Datenbank (11) mit dem ersten Speicherbereich (14) synchronisiert zu werden.

8. System nach einem der Ansprüche 1 bis 3, **dadurch gekennzeichnet, dass** der erste und/oder der zweite Berechtigungsschlüssel (16, 17) durch eine Operation in den Körper der Person (A) oder des Anwenders (B) eingesetzt werden.

9. System nach einem der Ansprüche 1 bis 3, **dadurch gekennzeichnet, dass** der erste und/oder der zweite Berechtigungsschlüssel (16, 17) Schmuckstücke sind.

10. System nach einem der Ansprüche 1 bis 3, **dadurch gekennzeichnet, dass** der erste und/oder der zweite Berechtigungsschlüssel (16, 17) Tätowierungen sind.

## Revendications

1. Système (1) comprenant une base de données (11), dans lequel dans la base de données sont mémorisées des informations personnelles (12) relatives à plusieurs personnes (A), lesdites informations (12) comprenant des informations (12) relatives audit état de santé des personnes, ainsi qu'un dispositif de sécurité (13) conçu pour protéger au moins des parties desdites informations contre un accès non autorisé, dans lequel ladite base de données (11) est divisée en un premier espace mémoire (14) et un deuxième espace mémoire (15), dans lequel ledit dispositif de sécurité (13) est conçu pour accorder à des utilisateurs autorisés (B) un accès audit premier espace mémoire (14) en fonction d'un premier niveau de sécurité et audit deuxième espace mémoire (15) en fonction d'un second niveau de sécurité, dans lequel dans ledit premier espace mémoire (14) sont mémorisées des informations générales (12a) relatives à l'état de santé de chaque dite personne (A), dans lequel dans ledit deuxième espace mémoire (15) sont mémorisées des informations spécifiques (12b) relatives à l'état de santé de chaque dite personne (A), dans lequel ledit dispositif de sécurité (13) est conçu pour demander une première clé d'autorisation (16) appartenant à ladite personne (A) en association avec une seconde clé d'autorisation (17) appartenant audit utilisateur autorisé (B) dans le but que l'accès audit premier espace mémoire (14) soit accordé audit utilisateur autorisé (B), dans lequel lesdites informations générales (12a) concernent des informations qui sont requises pour apporter une aide adéquate dans une situation grave, dans lequel ledit premier niveau de sécurité est conçu pour accorder à un utilisateur autorisé provenant d'un service médical public ou d'un corps d'assistance immédiate un accès auxdites informations générales (12a), dans lequel lesdites informations spécifiques (12b) concernent des informations confidentielles relatives à une action de soins de santé spécifique, dans lequel ledit second niveau de sécurité est conçu pour accorder à du personnel enregistré dans le secteur des soins un accès à des informations spécifiques sur ladite personne concernant l'action spéciale que ce même utilisateur utilise, dans lequel
ledit dispositif de sécurité (13) comprend des moyens (2) pour lire et vérifier lesdites première et seconde clés d'autorisation (16, 17), dans lequel lesdits moyens (2) comprennent une interface (2a) pour la communication avec une clé d'autorisation (16, 17), dans lequel ladite/lesdites première et/ou seconde clés d'autorisation (16, 17) sont/est quelque chose que possède une personne ou un utilisateur, **caractérisé en ce que**
ledit dispositif de sécurité (13) comprend une pluralité de différents moyens de lecture et de vérification (21, 22, 23, ..., 2n), chacun étant conçu pour lire et vérifier lesdites première et seconde clés d'autorisation (16, 17),
et **en ce que** ledit dispositif de sécurité (13), en réponse à une première clé d'autorisation (16) appartenant à une personne (A) quelconque en association avec une seconde clé d'autorisation (17) appartenant à un utilisateur (B) quelconque, est conçu pour accorder l'accès auxdites informations générales (12a) provenant dudit premier espace mémoire (14) concernant la personne (A) à laquelle appartient ladite première clé d'autorisation,
et **en ce que** lesdits moyens sont répartis et placés géographiquement pour être facilement accessibles par ledit utilisateur autorisé (B).

2. Système selon la revendication 1, **caractérisé en ce que** ledit dispositif de sécurité (13) est conçu pour déterminer un premier degré d'autorisation, qui est conçu pour donner à un utilisateur autorisé (B) l'autorisation de lire des informations provenant de ladite base de données (11), un deuxième degré d'autorisation, qui est conçu pour donner à un utilisateur autorisé (B) l'autorisation d'écrire des informations dans ladite base de données (11), ainsi qu'un troisième degré d'autorisation, qui est conçu pour donner à un utilisateur autorisé (B) l'autorisation de supprimer des informations de ladite base de données (11), **en ce qu'**un utilisateur autorisé (B) peut se voir accorder l'accès aux informations dans ladite base de données (11) en fonction d'une association adaptée desdits premier et second niveaux de sécurité et desdits premier, deuxième et troisième degrés, respectivement, d'autorisation, **en ce que** ledit système (1) comprend un journal (18), et **en ce que** chaque accès de ladite base de données (11), et chaque action entreprise lors d'un tel accès, soient enregistrés dans ledit journal (18) conjointement avec l'identité dudit utilisateur (B) ainsi que le moment de l'accès.

3. Système selon l'une quelconque des revendications précédentes, **caractérisé en ce que** ledit dispositif de sécurité est conçu pour accorder à ladite personne un accès auxdits premier et deuxième espaces mémoire (14, 15) au moyen de ladite première clé d'autorisation (16), et **en ce que** ledit accès est accordé au moins en fonction dudit premier degré d'autorisation.

4. Système selon l'une quelconque des revendications précédentes, **caractérisé en ce que** lesdits moyens (2) comprennent une interface (2a) pour la lecture d'informations biométriques et la fourniture d'une identification biométrique, et **en ce que** ladite/lesdites première et/ou seconde clés d'authentification (16, 17) sont/est une caractéristique unique d'un point de vue biométrique appartenant à ladite personne ou audit utilisateur.

5. Système selon l'une quelconque des revendications précédentes, **caractérisé en ce que** lesdits moyens (2) comprennent des outils d'analyse pour l'ADN, et **en ce que** ladite première clé d'autorisation est l'ADN de ladite personne.

6. Système selon la revendication 1, **caractérisé en ce que** lesdits moyens (23) sont mobiles, et **en ce que** lesdits moyens (23) sont conçus pour être transportés dans des véhicules d'urgence ou des véhicules de secours (4).

7. Système selon la revendication 6, **caractérisé en ce que** lesdits moyens (23) comprennent un troisième espace mémoire (5), **en ce qu'**une copie (12a') des informations générales (12a) mémorisées dans ledit premier espace mémoire (14) et appartenant à la personne (A) respective est mémorisée dans ledit troisième espace mémoire (5), **en ce qu'**un utilisateur autorisé (B) peut se voir accorder un accès auxdites informations générales (12a) par l'intermédiaire dudit troisième espace mémoire (5) dans une situation dans laquelle il est difficile ou impossible d'établir une communication avec ladite base de données (11), et **en ce que** ledit troisième espace mémoire (5) est conçu pour être synchronisé avec ledit premier espace mémoire (14) lors d'un contact entre lesdits moyens (23) et ladite base de données (11).

8. Système selon l'une quelconque des revendications 1 à 3, **caractérisé en ce que** ladite/lesdites première et/ou seconde clés d'autorisation (16, 17) sont/est introduite par une opération dans le corps de ladite personne (A) ou dudit utilisateur (B).

9. Système selon l'une quelconque des revendications 1 à 3, **caractérisé en ce que** ladite/lesdites première et/ou seconde clés d'autorisation (16, 17) sont/est un élément de bijouterie.

10. Système selon l'une quelconque des revendications 1 à 3, **caractérisé en ce que** ladite/lesdites première et/ou seconde clés d'autorisation (16, 17) sont/est un tatouage.
